(19) Europäisches Patentamt
European Patent Office
Office européen des brevets

(11) Publication number: 0 115 748
A1

(12) **EUROPEAN PATENT APPLICATION**

(21) Application number: 83830239.6

(51) Int. Cl.³: C 07 D 417/12, A 61 K 31/54

(22) Date of filing: 28.11.83

(30) Priority: 10.12.82 IT 4965682

(43) Date of publication of application: 15.08.84 Bulletin 84/33

(84) Designated Contracting States: AT BE CH DE FR GB LI LU NL SE

(71) Applicant: I.BIR.N. - Istituto Bioterapico Nazionale s.r.l., Via Grassi 9/11, I-00155 Roma (IT)

(72) Inventor: Frascatani, Domenico, Via Vittorio Grassi 9/11, Roma (IT)
Inventor: Gregorini, Anna Rosa, Via Vittorio Grassi 9/11, Roma (IT)

(74) Representative: Fiammenghi, Carlo et al, c/o Fiammenghi-Fiammenghi Via Quattro Fontane, No. 31, I-00184 Roma (IT)

(54) **Acetyl-piroxicam, preparation and use.**

(57) An anti-inflammatory, non-steroideus preparation of the class of the «Oxicam», obtained by acetylation of the 4-hydroxy-2-methyl-N-2 (Pyridil)-2H-1-2-benzothiazine-3-carboxyamide-1-1 dioxide which is a synthesis aromatic compound and the method for obtaining such a preparation.

EP 0 115 748 A1

0115748

# AN ANTI-INFLAMMATORY, NON-STEROIDEUS PREPARATION FOR THE TREATMENT OF THE ARTHRO-RHEUMATIC DISEASES AND METHOD FOR OBTAINING SUCH A PREPARATION

TITLE MODIFIED
see front page

The present invention relates to a non-steroideus preparation having anti-pyretic properties and which is obtained by acetylation of the 4-hydroxy-2-methyl-N-2 (pyridil)-2H-1-2-benzothiazine-3-carboxyamide 1-1--dioxide.

The starting product is known on the market as a product of the "Oxicam" series and is indicated with the trade-mark name: "Piroxicam".

It is known that the ratio between the chemical constitution of the pharmaceutic products and the pharmacologic action thereof is the main problem of the experimental pharmacology and that, according to what E. Adami (Pharmacology and Pharmacotherapy) has written, the aromatic compounds have often an antipyretic and antiseptic action, as, for instance, the phenol, the salicylic acid, the antipyrine and so on.

This invention starts from the presupposition that, if an OH-hydroxyl group is introduced in the aliphatic chains, it increases generally the pharmacologic activity thereof; in contrast, if it is introduced in the aromatic series, the toxicity is increased.

The acetylation of an active principle of such a chemical

constitution thus leads to less toxic compounds. In fact the acetamide is less toxic than the ammonia, as well as acetanilide is less toxic than the aniline, and so on. In accordance with what A. Bonanni (Experimental Pharmacology and Toxilogy) has described, the acetylation of an alcoholic function increases or modifies the pharmacologic effect; the acetylcholine is more active than the choline, the acetyl-salicilic acid is more active than the salicilic acid; as a further example, can be cited this latter: i.e. the morphine, after acetylation leads to the diacetylmorphine (Heroin). If this compound is introduced in a living organism, it is easily saponified and transformed into morphine at a nascent state, which, as has been pharmacologically proved, has a more intense analgesic and narcotic activity. In addition, in so far as the salicilic acid is concerned, -which has been diffusely used from ten-year periods in the treatment of the rheumatism, the rheumatoid arthritis, the osteo-arthrosis, the spondilite and the muscular-skeletal diseases, the acetylation (acetyl-salicilic acid) has produced compounds of superior activity, thus permitting either to decrease the administration dose, or to reduce the collateral effects.

The present invention thus aims at obtaining a product less toxic than the product known under the trade-mark "Piroxicam", by providing the acetylation of the OH-group in the formula of the 4-hydroxi-2-methyl-N-2 pyridil-2H-

1-2 benzothiazine-3-carboxyamide-1-1-dioxide of the compound already on the market.

The utility of obtaining the acetyl-compound of the "Piroxicam" is due to the purpose of realizing such a therapeutic means which has at the same time a less toxicity and less undesired secondary effects together with a pharmacologic anti-inflammatory action of equal effectiveness in accordance with what has been stated in the preamble of the description.

The method consists in carrying out a synthesis of the starting product by means of an addition of acetic anhydride and sodium acetate, heating to about 30-40°C under stirring until to attain a complete solubilizing, then heating the mixture until an incipient boiling, and a flowing back for about two hours, the whole being then poured in cold water. Then the mixture is allowed to rest for about 24 hours, then carrying out a filtering and washing. The filtering residue is dissolved in ethanol and is evaporated until a new product in the form of crystals is obtained. This last operation can be repeated.

As a variant of such a method provision has been made of substituting a portion of the acetic anhydride with acetic acid.

Two qualitative and quantitative examples of the method are now given.

<u>Example 1</u>

10 parts by weight of 4-hydroxy-2-methyl-N-2 pyridil-2H--1-2 benzothiazine-3-carboxyamide-1-1-dioxide are added to 40 parts by weight of sodium acetate and are mixed with an addition of 120 parts by weight of acetic anhydride, stirring at a temperature of about 40°C until solubilizing.

Then the mixture is heated until an incipient boiling and a flowing back for two hours and then it is poured into distilled water. The mixture is then heated for some minutes in order that the acetic anhydride can be decomposed and then is cooled. The mixture is allowed to deposit for 24 hours; then it is filtered and the filtrate is washed with distilled water. The residue on the filter is dissolved at 95°C by means of warm ethanol as it will be sufficient: then it is hot evaporated until incipient crystallisation thereof. Distilled water is added under stirring and the obtained precipitate is recovered by a filtering and at last it is dried under an air flow. Then it is recrystallized, carrying out a cooling at the ambient temperature with an addition of anhydrous ethanol and a heating until boiling in the proportions of one part of substance per 10 parts of ethanol.

Example 2

10 parts by weight of 4-hydroxy-2-methyl-N-2 pyridil-2 H--1-2-benzothyazine-3-carboxyamide-1-1-dioxide are dissolved in 20 parts by weight of glacial acetic acid; 30 parts by weight of sodium acetate and 100 parts by weight of acetic anhydride are then added under stirring.

The mixture is heated until solubilizing at 30-40°C until an incipient boiling and is flowed back for two hours. Then it is poured into distilled water; then it is heated for some minutes so as to decompose the acetic anhydride and then it is cooled. Afterwards the procedure as in the Example 1 is followed.

The characteristics of the product which is so obtained, are the following ones:

- Needle-shaped, early white, translucent crystals, insoluble in water at ambient temperature.
- Product which is soluble in ethanol, more in warm than in cold conditions, in chloroform, benzene and acetic acid.
- Chemical name (according the JUPAC Rules): N-2(pyridil)-4-oxy-acetyl-2 methyl-2H-1-2-benzothiazine-3-carboxy-amide-1-1-dioxide.
- Chemical formula:

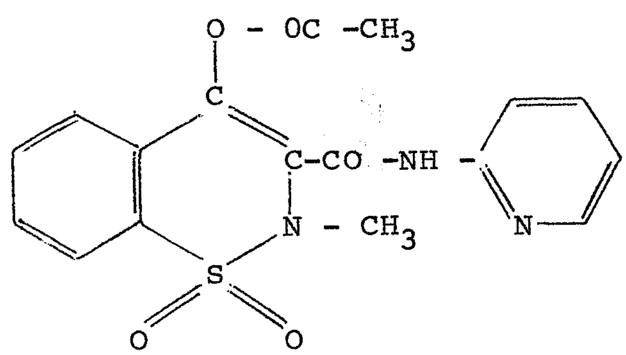

- Brute formula: $C_{17}H_{15}N_3O_5S$
- Molecular weight: 373.37
- Product which remains stable in the time at ambient temperature and which is not hygroscopic and does not

give off any scent of acetic acid.

- P.F.= 122 C (crystalized by methanol)

- Drying loss: at 60°C under vacuum for 3 hours it does not lose more than 0.5% of its weight.

- Reaction: about 250 mg are mixed, under an active stirring, with 100 ml of $H_2O$; then a filtration is carried out. The pH of the filtrate is = 6.

As identification and differentiations of the product of the invention, it is to be noted that:

1) No reaction of the product in question takes place with ferric chloride, while reactions have been found in the starting product "Piroxicam" owing to the presence of the phenolic group;

2) In so far as U.V. spectrophotometry is concerned, it was found that a 0.001% methanolic solution (mcq 10/ml) which has been examined with a spectrophotometer against a methanol white shows two absorption maxima at nm 233 and nm 280 and a minimum at nm 245. The starting product employed for the synthesis has the maxima at nm 240 and the minimum at nm 265 respectively.

CLAIMS

1. An anti-inflammatory, non steroideus preparation having the chemical name: N-2 (pyridil)-4-oxy-acetyl-2--methyl-2H-1-2-benzothiazine-3-carboxyamide-1-1-dioxide (in accordance with the JUPAC Rules) and the chemical formula:

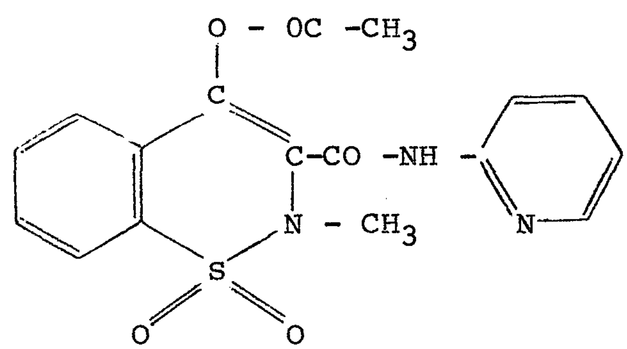

2. A method for obtaining the acethyl compound according to claim 1, by acetylation of the OH-group of the starting product: 4-hydroxy-2-methyl-N-2 (pyridil)-2H-1--2-benzothiazine-3-carboxyamide-1-1-dioxide.

3. A method according to claim 2, wherein 10 parts by weight of the starting product are added to 40 parts by weight of sodium acetate to which 120 parts of acetic anhydride are added at a temperature of 30°C-40°C and mixed, under mixing, and the mixture is then stirred until the complete solubilizing, then heating the solution until an incipient boiling and then flowing back for about two hours, the whole being then poured in cold distilled water, afterwards the mixture is heated for

some minutes so as to obtain the decomposition of the acetic anhydride and then it is cooled and is allowed to deposit for about 24 hours, afterwards it is filtered and the filtrate is washed with distilled water, the residue on the filter being then dissolved in ethanol heated at about 95°C for a sufficient time period, then a warm evaporation is carried out until an incipient crystallization, afterwards distilled water is added and the precipitate is recovered by filtering, then drying the precipitate in an air flow, the dry product, which is so obtained, being then recrystallized with an addition of ethanol in the proportions of one part of the product per 10 parts of ethanol, thus obtaining the recrystallisation of the filtrate by the cooling thereof at ambient temperature.

4. A method according to claims 1 and 2, wherein, for carrying out the acetylation, 20 parts by weight of glacial acetic acid are added to 10 parts by weight of the starting product, then 30 parts by weight of sodium acetate and 100 parts of acetic anhydride under stirring, are added, heating the mixture until a complete solubilizing at 30-40°C, aftewards, in order to carry out the separation of the product, the procedure described in claim 3 is followed.

European Patent Office

# EUROPEAN SEARCH REPORT

0115748
Application number

EP 83 83 0239

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
| --- | --- | --- | --- |
| X | EP-A-0 057 059 (PFIZER)<br>* Claims *<br>--- | 1-4 | C 07 D 417/12<br>A 61 K 31/54 |
| P,X | EP-A-0 085 866 (CHIESI FARMACEUTICI S.p.A.)<br>* Claims *<br>----- | 1-4 | |
| | | | **TECHNICAL FIELDS SEARCHED (Int. Cl. 3)** |
| | | | C 07 D 417/00<br>A 61 K 31/00 |

The present search report has been drawn up for all claims

| Place of search | Date of completion of the search | Examiner |
| --- | --- | --- |
| THE HAGUE | 08-03-1984 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82